# FASCICULE DE BREVET EUROPEEN

(11) **EP 1 335 694 B1**
(45) Date de publication et mention de la délivrance du brevet: **20.09.2006**
(21) Numéro de dépôt: 01993448.8
(22) Date de dépôt: 06.11.2001
(51) Int. Cl.: A61Q 5/10, A61K 8/18

(54) **COMPOSITION DE TEINTURE DIRECTE POUR FIBRES KERATINIQUES COMPRENANT UN POLYURETHANE ASSOCIATIF CATIONIQUE**
DIREKTFÄRBEMITTEL FÜR KERATINISCHE FASERN ENTHALTEND EIN KATIONISCHES ASSOZIATIVES POLYURETHAN
DIRECT DYEING COMPOSITION FOR KERATINOUS FIBRES COMPRISING A CATIONIC ASSOCIATIVE POLYURETHANE

(30) Priorité: 08.11.2000 FR 0014322
(43) Date de publication de la demande: 20.08.2003
(73) Titulaire: L'ORÉAL, 75008 Paris (FR)
(72) Inventeur: COTTARD, François, F-92400 Courbevoie (FR); DE LA METTRIE, Roland, F-78110 Le Vésinet (FR)
(74) Mandataire: Wattremez, Catherine
(86) Numéro de dépôt international: PCT/FR2001/003427
(87) Numéro de publication internationale: WO 2002/038115

(56) Documents cités:
- US-A- 5 478 562
- US-A- 5 807 957
- US-A- 5 876 463

## Description

La présente invention concerne une composition de teinture directe des fibres kératiniques, en particulier des fibres kératiniques humaines et plus particulièrement les cheveux, comprenant au moins un colorant direct et au moins un polyuréthane associatif cationique.

Il est connu de teindre les fibres kératiniques et en particulier les cheveux humains, avec des compositions de teinture contenant des colorants directs, en particulier des colorants benzéniques nitrés, des colorants azoïques acides, des colorants azoïques cationiques, des colorants anthraquinoniques, des colorants naturels.
Ces colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.

Pour localiser le produit de coloration à l'application sur les cheveux afin qu'il ne coule pas sur le visage ou en dehors des zones que l'on se propose de teindre, on a jusqu'ici eu recours à l'emploi d'épaississants traditionnels tels que l'acide polyacrylique réticulé, les hydroxyéthylcelluloses, les cires ou encore à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Cependant, la demanderesse a constaté que les systèmes épaississants mentionnés ci-dessus ne permettaient pas d'obtenir des nuances puissantes et chromatiques de faibles sélectivités et de bonnes ténacités tout en assurant un bon état cosmétique à la chevelure traitée. Par ailleurs, elle a également constaté que les compositions tinctoriales prêtes à l'emploi contenant le ou les colorants directs, et en outre les systèmes épaississants de l'art antérieur ne permettaient pas une application suffisamment précise sans coulures ni chutes de viscosité dans le temps.

Or, après d'importantes recherches menées sur la question, la Demanderesse vient maintenant de découvrir qu'il est possible d'obtenir des compositions de teinture directe qui ne coulent pas et restent donc bien localisées au point d'application, et qui permettent aussi d'obtenir des nuances puissantes et chromatiques (lumineuses) avec de faibles sélectivités et de bonnes ténacités vis à vis des agents chimiques ( shampooing, permanentes ...) ou naturels (lumière, transpiration ...) tout en apportant aux cheveux de bonnes propriétés cosmétiques si on introduit (i) soit dans la composition contenant au moins un colorant direct [ou composition A1], soit (ii) dans la composition oxydante [ou composition B1] utilisée pour la coloration directe éclaircissante, ou (iii) dans les deux compositions à la fois, une quantité efficace d'un polyuréthane associatif cationique.

Ces découvertes sont à la base de la présente invention.

La présente invention a ainsi pour objet une composition de teinture directe pour fibres kératiniques, en particulier pour fibres kératiniques humaines et plus particulièrement les cheveux, comprenant, dans un milieu approprié pour la teinture, au moins un colorant direct, qui est caractérisée par le fait qu'elle comprend en outre au moins un polyuréthane associatif cationique.

Un autre objet de l'invention porte sur une composition prête à l'emploi pour la teinture des fibres kératiniques qui comprend au moins un colorant direct et au moins un polyuréthane associatif cationique et un agent oxydant.
Par "composition prête à l'emploi", on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

L'invention vise également un procédé de teinture directe des fibres kératiniques, en particulier pour fibres kératiniques humaines et plus particulièrement les cheveux, consistant à appliquer sur les fibres une composition comprenant, dans un milieu approprié pour la teinture, au moins un colorant direct et au moins un polyuréthane associatif cationique.
L'invention vise aussi un procédé de teinture directe éclaircissante des fibres kératiniques, en particulier pour fibres kératiniques humaines et plus particulièrement les cheveux, consistant à appliquer sur les fibres un mélange extemporané d'une composition A1 comprenant, dans un milieu approprié pour la teinture, au moins un colorant direct, et d'une composition B1 comprenant au moins un agent oxydant, la composition A1 et/ou la composition B1 comprenant au moins un polyuréthane associatif cationique.

L'invention a également pour objet des dispositifs pour la teinture directe et la teinture directe éclaircissante des fibres kératiniques humaines et plus particulièrement les cheveux, ou " kits " à deux compartiments.
Un dispositif à deux compartiments pour la teinture directe selon l'invention comprend un premier compartiment renfermant, dans un milieu approprié pour la teinture, au moins un colorant direct et un deuxième compartiment renfermant au moins un polyuréthane associatif cationique.
D'autres dispositifs à 2 compartiments pour la teinture directe éclaircissante selon l'invention comprennent un compartiment qui renferme une composition A1 comprenant, dans un milieu approprié pour la teinture, au moins un colorant direct, et un autre compartiment qui renferme une composition B1 comprenant, dans un milieu approprié pour la teinture, un agent oxydant, au moins un polyuréthane associatif cationique étant présent dans la composition A1 ou la composition B1, ou dans chacune des compositions A1 et B1.

Mais d'autres caractéristiques, aspects, objets et avantages de l'invention apparaîtront encore plus clairement à la lecture de la description et des exemples qui suivent.

Les polymères associatifs sont des polymères dont les molécules sont capables, dans le milieu de formulation, de s'associer entre elles ou avec des molécules d'autres composés.
Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou avec d'autres molécules.

### Polyuréthanes associatifs cationiques

Les polyuréthanes associatifs cationiques selon la présente invention sont plus particulièrement choisis parmi les polyuréthanes amphiphiles associatifs cationiques, hydrosolubles ou hydrodispersibles.
Le terme "hydrosoluble" ou "soluble dans l'eau" concernant les polyuréthanes associatifs de la présente invention signifie que ces polymères ont une solubilité dans l'eau à température ambiante au moins égale à 1 % en poids, c'est-à-dire que jusqu'à cette concentration, aucun précipité ne peut être détecté à l'oeil nu et la solution est parfaitement limpide et homogène.

On entend par polyuréthanes "hydrodispersibles" ou "dispersibles dans l'eau" des polymères qui, lorsqu'on les met en suspension dans l'eau, forment spontanément des globules ayant une taille moyenne, mesurée par diffusion de la lumière sur un appareil de type Coulter, comprise entre 5 nm et 600 nm, et en particulier entre 5 nm et 500 nm.

La famille de polyuréthanes associatifs cationiques selon l'invention a été décrite par la demanderesse dans la demande de brevet français N°-0009609; elle peut être représentée par la formule générale (Ia) suivante :

R-X-(P)ₙ-[L-(Y)ₘ]ᵣ-L'-(P')ₚ-X'-R' (Ia)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ; la molécule contenant au moins une fonction amine protonée ou quaternisée et au moins un groupement hydrophobe.

Dans un mode de réalisation préféré des polyuréthanes de la présente invention, les seuls groupements hydrophobes sont les groupes R et R' aux extrémités de chaîne.

Une famille préférée de polyuréthanes associatifs cationiques est celle correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (la) ci-dessus dans laquelle :
R et R' représentent tous les deux indépendamment un groupement hydrophobe, X,
X' représentent chacun un groupe L", n et p valent 0, et L, L', L", Y et m ont la signification indiquée ci-dessus.
Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (Ia) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.
Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.
Lorsque X et/ou X' désignent un groupement comportant une amine tertiaire ou quaternaire, X et/ou X' peuvent représenter l'une des formules suivantes : 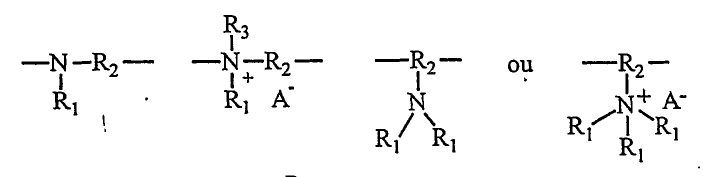 pour X 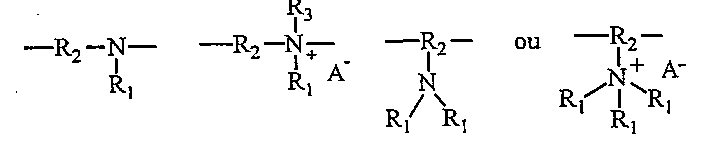 pour X' dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 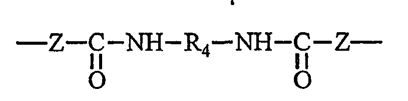 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 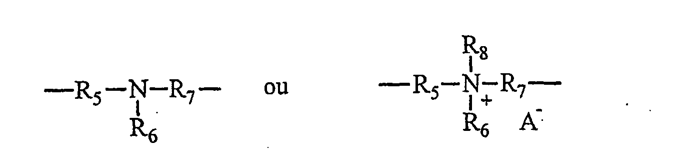 ou 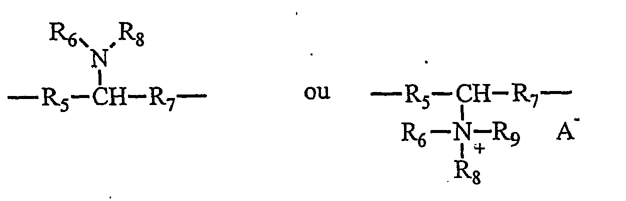 ou 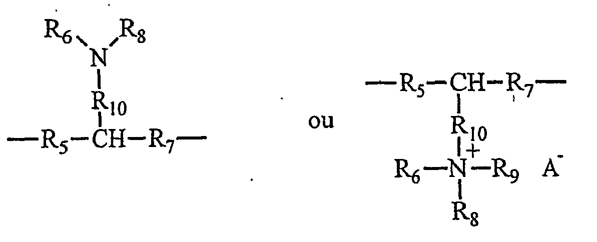 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.
A titre d'exemple, on peut citer, lorsqu'il ne s'agit pas de polymères, l'éthylèneglycol, le diéthylèneglycol et le propylèneglycol.
Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (Ia) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (Ia) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine.
Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (Ia) est un diisocyanate correspondant à la formule :

O=C=N-R₄-N=C=O

dans laquelle R₄ est défini plus haut.
A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (Ia) est un composé hydrophobe destiné à former les groupes hydrophobes terminaux du polymère de formule (Ia).

Ce composé est constitué d'un groupe hydrophobe et d'une fonction à hydrogène labile, par exemple une fonction hydroxyle, amine primaire ou secondaire, ou thiol.
A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du pqlybutadiène hydrogéné - hydroxyle.
Le groupe hydrophobe du polyuréthane de formule (Ia) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
A titre d'exemple, on peut citer, lorsqu'il ne s'agit pas de polymères, l'éthylèneglycol, le diéthylèneglycol et le propylèneglycol.
Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (Ia) est facultatif. En effet, les motifs à fonction amine quaternaire ou protonée peuvent suffire à apporter la solubilité ou l'hydrodispersibilité nécessaire pour ce type de polymère dans une solution aqueuse.
Bien que la présence d'un groupe Y hydrophile soit facultative, on préfère cependant des polyuréthanes associatifs cationiques comportant un tel groupe.
Lesdits polyuréthanes associatifs cationiques sont hydrosolubles ou hydrodispersibles.

Les polyuréthanes associatifs cationiques sont utilisés de préférence en une quantité pouvant varier d'environ 0,01 à 10% en poids du poids total de la composition de teinture prête à l'emploi. Plus préférentiellement, cette quantité varie d'environ 0,1 à 5% en poids.

Les colorants directs utilisables selon l'invention sont choisis de préférence parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants:
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoiques cationiques décrits dans les demandes de brevets WO 95/15144, WO-95/01772 et EP-714954 dont le contenu fait partie intégrante de l'invention.
On peut également citer parmi les colorants directs azoïques les colorants suivants, décrits dans le COLOUR INDEX INTERNATIONAL 3^{e} édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.
On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényi-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Plus particulièrement, les compositions selon l'invention peuvent comprendre en outre au moins un polymère substantif amphotère ou cationique différent des polyuréthanes associatifs cationiques de l'invention.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 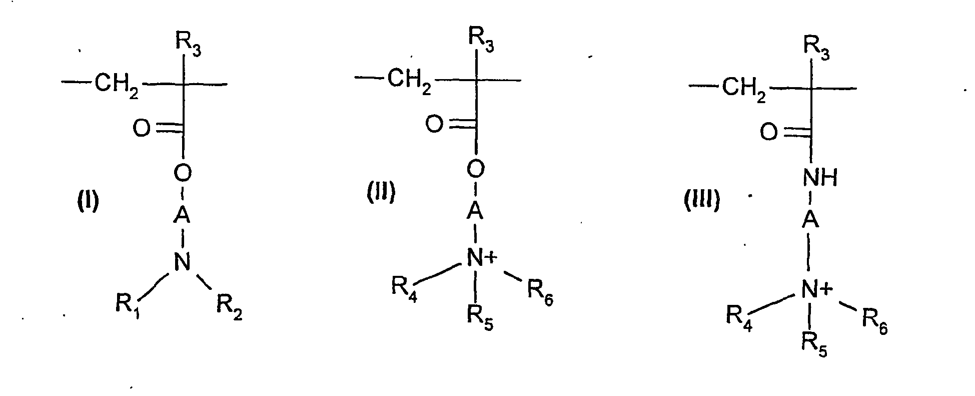 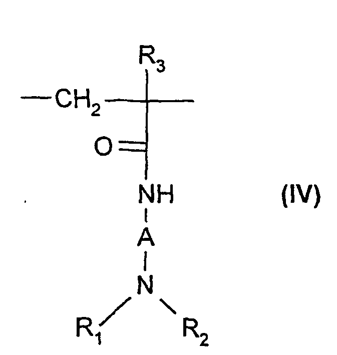 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
      Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
      - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
      - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
      - le copolymère d'acrylamide et de méthosulfate de méthacryloyloxyéthyltriméthylammonium vendu sous la dénomination RETEN par la société HERCULES,
      - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
      - les terpolymères méthacrylate de diméthyl amino éthyle/ vinylcaprolactamel vinylpyrrolidone tel que le produit vendu sous la dénomination GAFFIX VC 713 par la société ISP,
      - les copolymère vinylpyrrolidone / méthacrylamidopropyl dimethylamine commercialisés notamment sous la dénomination STYLEZE CC 10 par ISP,
      - et les copolymères vinylpyrrolidone / méthacrylamide de diméthylaminopropyle quaternisés tel que le produit vendu sous la dénomination "GAFQUAT HS 100" par la société ISP.
**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quatemisées. De tels polymères sont notamment décrits dans les brevets français 2.252.840 et 2.368.508 .
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 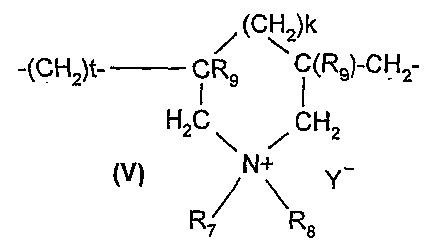 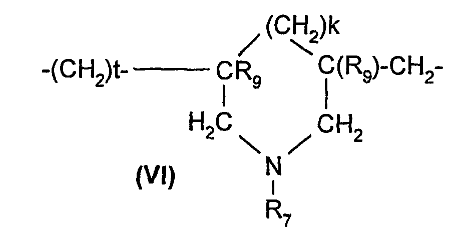 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
**(10)** Le polymère de diammonium quaternaire contenant des motifs récurrents répondant à la formule : 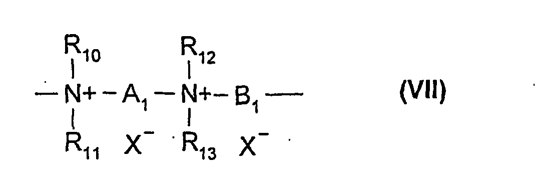 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
      On peut utiliser plus particulièrement les polymères qui sont constitués de motifs récurrents répondant à la formule (VIII) suivante: 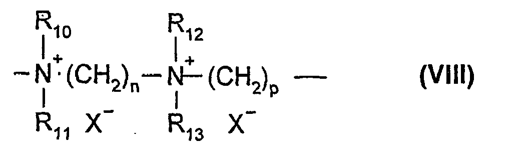 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
**(11)** Les polymères de polyammonium quaternaire constitués de motifs récurrents de formule (IX) : 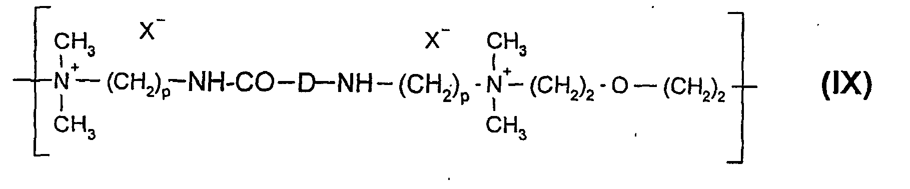 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
**(12)** Les polymères quaternaires de vinylpyrrolidone et de vinylimidazole tels que par exemple les produits commercialisés sous les dénominations Luviquat FC 905, FC 550 et FC 370 par la société B.A.S.F.
**(13)** Les polyamines comme le Polyquart H vendu par HENKEL, référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁₋C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 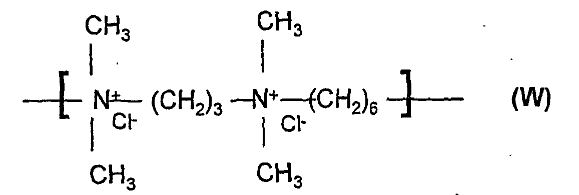 et notamment ceux dont le poids moléculaire, déterminé par chromatographie par perméation de gel, est compris entre 9500 et 9900; 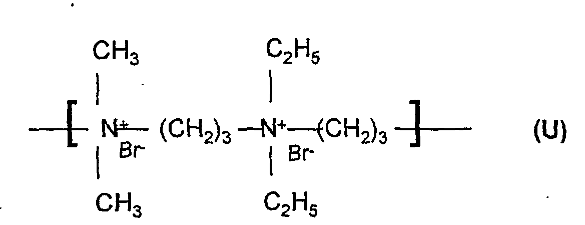 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant.au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylateslbuty/aminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 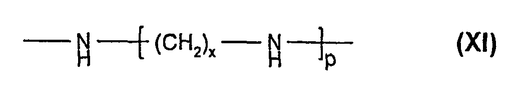 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 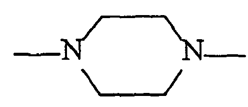
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 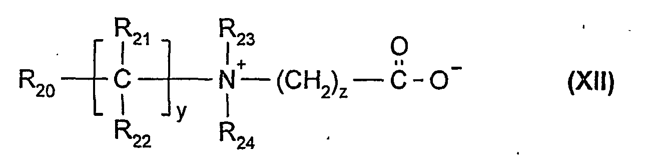 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 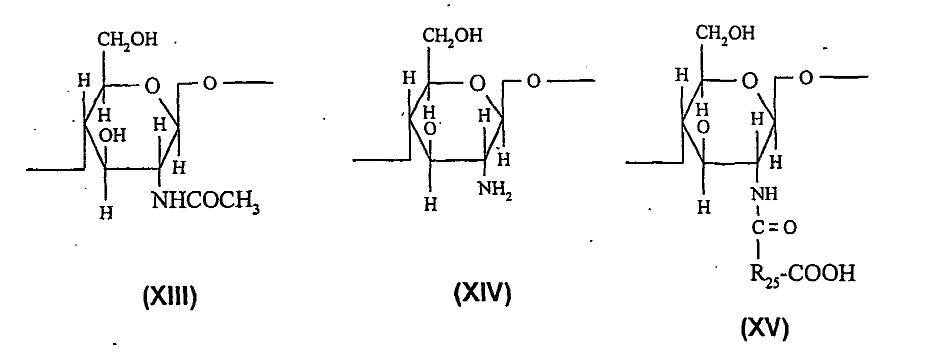 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 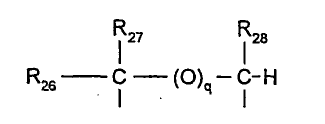 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 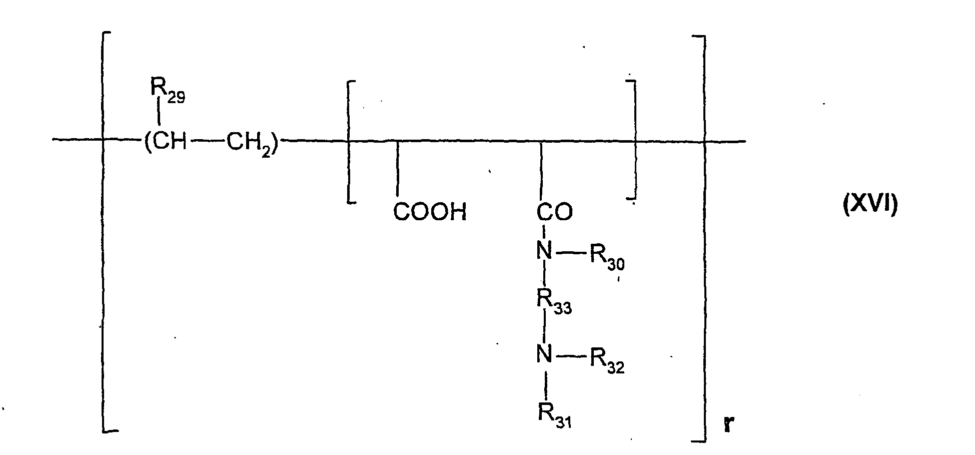 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XVII)**

      où D désigne un radical 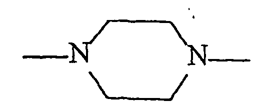 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XVIII)**

      où D désigne un radical 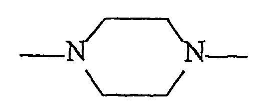 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs. Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆₋C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

### (iv) Tensioactifs cationiques

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Les compositions selon l'invention peuvent également comprendre d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique et les polymères associatifs ioniques ou non ioniques tels que les polymères commercialisés sous les appellations PEMULEN TR1 ou TR2 par la société GOODRICH, SALCARE SC90 par la société ALLIED COLLOIDS, ACULYN 22, 28, 33, 44, ou 46 par la société ROHM & HAAS et ELFACOS T210 et T212 par la société AKZO.

Ces épaississants d'appoint peuvent représenter de 0,01 à 10% en poids du poids total de la composition.

Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

La composition A peut encore comprendre une quantité efficace d'autres agents, par ailleurs antérieurement connus en coloration directe, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, des polymères associatifs autres que ceux de l'invention, et en particulier des polyuréthanes polyéthers associatifs non-ioniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi avec agent oxydant, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi [composition sans oxydant prête à l'emploi ou composition résultant du mélange de la composition tinctoriale A1 et de la composition oxydante B1], est généralement compris entre les valeurs 2 et 12. Il est de préférence compris entre 3 et 11, et peut être ajusté à la valeur désirée au moyen d'agents acidifiants ou alcalinisants bien connus de l'état de la technique en teinture des fibres kératiniques.
Plus préférentiellement lorsque la composition contient un agent oxydant pour l'éclaircissement des fibres, le pH du mélange prêt à l'emploi est supérieur à 7 et encore plus préférentiellement supérieur à 8.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante : 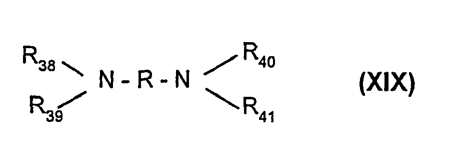 dans laquelle R est un reste propylène éventuellement substitué par un groupement hydroxyle ou un radical alkyle en C₁-C₄ ; R₃₈, R₃₉, R₄₀ et R₄₁, identiques où différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition sans oxydant prête à l'emploi A, ou la composition réalisée extemporanément au moment de l'emploi à partir des compositions A1 et B1 décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Une variante de ce procédé consiste à prendre une composition A' comprenant au moins un colorant direct mais sans polyuréthane associatif cationique, une composition A" comprenant au moins un polymère polyuréthane associatif cationique et à mélanger au moment de l'emploi ces compositions A', A" avec la composition B oxydante, puis à appliquer et laisser agir le mélange comme précédemment.

Des exemples concrets illustrant l'invention sont indiqués ci-après, sans pour autant présenter un caractère limitatif.

### EXEMPLE 1 :

On a préparé les compositions suivantes :
(exprimées en grammes)

**Composition oxydante :**

| | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée qsp | 100 |

**Composition colorante :**

| | A | B |
|---|---|---|
| alcools gras oxyéthylénés | 32,5 | 32,5 |
| Acide oléique | 2 | 2 |
| Alcool oléique | 1,8 | 1,8 |
| Amide gras | 4 | 4 |
| Glycérine | 3 | 3 |
| Polymère cationique de formule (W) en solution à 60% dans l'eau | 1,2MA* | 1,2MA* |
| Merquat 280 | 2 | 2 |
| Agent séquestrant | qs | qs |
| Réducteur | qs | qs |
| Ammoniaque (20% NH3) | 8 | 8 |
| Diamino-1,4-nitro-2-benzène | 0,6 | 0,6 |
| Polymère 1 | 0,3MA* | |
| Polymère 2 | | 0,3MA* |
| Eauqsp | 100g | 100g |

| | | |
|---|---|---|
| MA* = Matière Acitve | | |

Le polymère 1 est le polymère suivant :
C₁₈H₃₇-O-CONHR₄NHCO-O-(CH₂)₂-N⁺(CH₃)(CH₃)-(CH₂)₂-O-CONHR₂NHCO-O(POE)O-CONHR₂NHCO-O-(CH₂)₂₋N⁺(CH₃)(CH₃)-(CH₂)₂-O-CONHR₄NHCO-OC₁₈H₃₇
avec :
R₄ = méthylènedicyclohexyle
contre ion : CH₃SO₄⁻

II est synthétisé à partir des réactifs suivants :

| | |
|---|---|
| C₁₈H₃₇OH 2 | moles |
| Méthylènedicyclohexyldiisocyanate | 4 moles |
| Polyéthylèneglycol | 1 mole |
| N-méthyléthanolamine | 2 moles |
| Agent quaternisant (CH₃)₂SO₄ 2 | moles |

Le polymère 2 est le polymère suivant :
C₁₈H₃₇N⁺(CH₃)(CH₃)-(CH₂)₂-O-CONHR₄NHCO-O(POE)O-CONHR₄NHCO-O(CH₂)₂-N⁺(CH₃)(CH₃)C₁₈H₃₇
avec :
R₄ = méthylènedicyclohexyle
Contre ion : Cl⁻

Il est synthétisé à partir des réactifs suivants :

| | |
|---|---|
| Méthylènedicydohexyldiisocyanate | 2 moles |
| Polyéthylèneglycol | 1 mole |
| N,N-diméthyléthanolamine | 2 moles |
| Agent quaternisant C₁₈H₃₇OH | 2 moles |

Les compositions colorantes ont été mélangées, au moment de l'emploi, dans un bol en plastique et pendant 2 minutes, à la composition oxydante donnée ci-dessus, à raison de 1 partie de composition colorante pour 1,5 parties de composition oxydante.
On a appliqué les mélanges obtenus sur des mèches de cheveux naturels à 90% de blancs et on a laissé poser 30 minutes. On a ensuite rincé les mèches à l'eau, on les a lavées au shampooing standard, à nouveau rincées à l'eau, puis séchées et démélées.
Les cheveux ont été teints dans les deux cas dans une nuance rouge puissante.

### EXEMPLE 2

On a préparé la composition suivante :
(exprimées en grammes)

| | |
|---|---|
| alcools gras oxyéthylénés | 32,5 |
| Acide oléique | 2 |
| Alcool oléique | 1,8 |
| Amide gras | 4 |
| Glycérine | 3 |
| Basic Red 76 | 0,4 |
| Polymère 1 | 0,3 MA* |
| Amino-2-méthyl-2-propanol-1 qs | pH 8,5 |
| Eau qsp | 100 |

| | |
|---|---|
| MA* = Matière Active | |

Cette composition a été appliquée trente minutes sur des cheveux gris à 90% de blancs. Après rinçage et séchage les cheveux ont été colorés en rouge intense.

## Revendications

1. Composition de teinture directe pour fibres kératiniques, en particulier pour fibres kératiniques humaines et plus particulièrement les cheveux, comprenant dans un milieu approprié pour la teinture, au moins un colorant direct, **caractérisée par le fait qu'**elle comprend en outre au moins un polyuréthane associatif cationique.

2. Composition selon la revendication 1 **caractérisée par le fait que** le polyuréthane associatif cationique est de formule (Ia) suivante :
R-X-(P)ₙ-[L-(Y)ₘ]ᵣ-L'-(P')ₚ-X'-R' (Ia)
dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ; la molécule contenant au moins une fonction amine protonée ou quaternisée et au moins un groupement hydrophobe.

3. Composition selon la revendication 2 **caractérisée par** le fait R et R' représentent tous les deux indépendamment un groupement hydrophobe, X, X' représentent chacun un groupe L", n et p valent entre 1 et 1000 et L, L', L", P, P', Y et m ont la signification indiquée dans la revendication 2.

4. Composition selon la revendication 2, **caractérisée par le fait que** R et R' représentent tous les deux indépendamment un groupement hydrophobe, X et X' représentent tous les deux indépendamment un groupement comportant une amine quaternaire, n et p valent zéro, et L, L', Y et m ont la signification indiquée dans la revendication 2.

5. Composition selon l'une quelconque des revendications 2 à 4, **caractérisée par le fait que** R et R' représentent un radical ou un polymère à chaîne hydrocarbonée, saturée ou non, linéaire ou ramifiée, dans laquelle un ou plusieurs des atomes de carbone peut être remplacé par un hétéroatome choisi parmi S, N, O et P, ou à chaîne siliconée ou perfluorée.

6. Composition selon l'une quelconque des revendications 2 à 5, **caractérisée par le fait que** X et X' représentent l'une des formules : 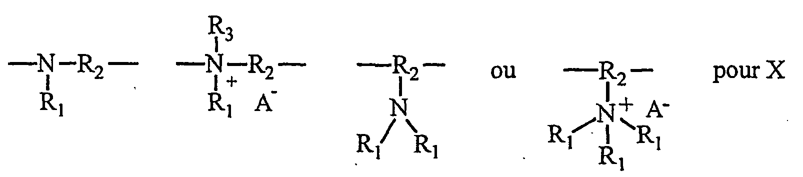 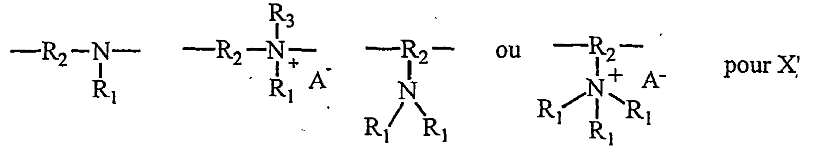 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

7. Composition selon l'une quelconque des revendications 2 à 6, **caractérisée par le fait que** les groupements L, L' et L", identiques ou différents, représentent la formule : 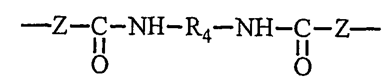 dans laquelle :
Z représente -O-, -S- ou -NH- ; et
R₄ représente un radical alkylène ayant de 1 à 20 atomes de carbone, linéaire ou ramifié, comportant ou non un cycle saturé ou non saturé, un radical arylène, un ou plusieurs des atomes de carbone pouvant être remplacé par un hétéroatome choisi parmi N, S, O et P.

8. Composition selon l'une quelconque des revendications 2 à 7, **caractérisée par le fait que** les groupements P et P', identiques ou différents, représentent au moins l'une des formules suivantes : 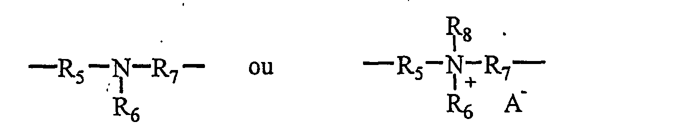 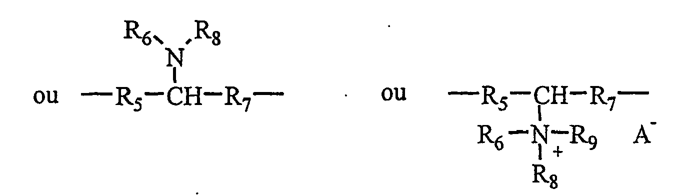 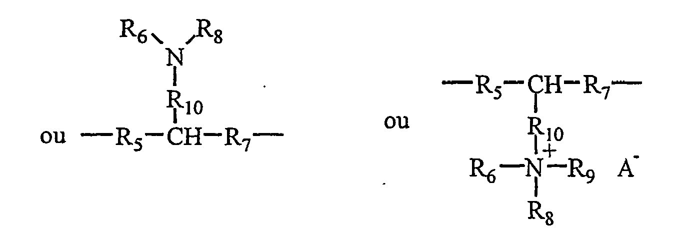 dans lesquelles :
R₅ et R₇ ont les mêmes significations que R₂ ;
R₆, R₈ et R₉ ont les mêmes significations que R₁ et R₃ ;
R₁₀ représente un groupe alkylène, linéaire ou ramifié, éventuellement insaturé et pouvant contenir un ou plusieurs hétéroatomes choisis parmi N, O, S et P, et
A⁻ est un contre-ion physiologiquement acceptable.

9. Composition selon l'une quelconque des revendications 2 à 8, **caractérisée par le fait que** Y représente un groupe dérivé d'éthylèneglycol, de diéthylèneglycol ou de propylèneglycol, ou un groupe dérivé d'un polymère choisi parmi les polyéthers, les polyesters sulfonés et les polyamides sulfonés.

10. Composition selon l'une quelconque des revendications précédentes, **caractérisée par le fait que** les polyuréthanes associatifs cationiques ont une masse moléculaire moyenne en nombre comprise entre 400 et 500000, de préférence entre 1000 et 400000 et en particulier entre 1000 et 300000.

11. Composition selon l'une quelconque des revendications précédentes, **caractérisée par le fait que** les polyuréthanes associatifs cationiques sont utilisés en une quantité variant de 0,01 à 10% en poids du poids total de la composition.

12. Composition selon la revendication 11, **caractérisée par, le fait que** les polyuréthanes associatifs cationiques sont utilisés en une quantité variant de 0,1 à 5% en poids du poids total de la composition.

13. Composition selon l'une quelconque des revendications précédentes, **caractérisée par le fait que** le colorant direct est choisi parmi les colorants directs nitrés benzéniques neutres, acides ou cationiques, les colorants directs azoïques neutres, acides ou cationiques, les colorants directs quinoniques et en particulier anthraquinoniques, neutres, acides ou cationiques, les colorants directs aziniques, les colorants directs triarylméthaniques, les colorants directs indoaminiques et les colorants directs naturels.

14. Composition selon l'une quelconque des revendications précédentes, **caractérisée par le fait que** le ou les colorants directs sont présents dans des concentrations allant de 0,001 à 20% et de préférence de 0,005 à 10% en poids par rapport au poids total de la composition.

15. Composition selon l'une quelconque des revendications précédentes, **caractérisée par le fait qu'**elle contient en outre au moins un polymère substantif amphotère ou cationique différent des polyuréthanes associatifs cationiques décrits à l'une quelconque des revendications 2 à 12.

16. Composition selon la revendication 15, **caractérisée par le fait que** le polymère cationique est un polymère de polyammonium quaternaire constitué de motifs récurrents répondant à la formule (W) suivante :

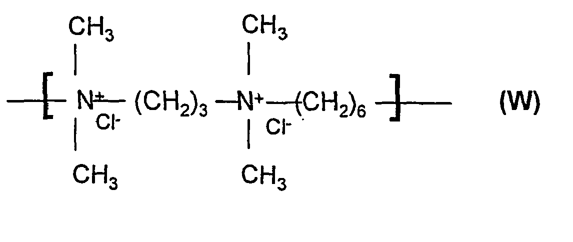

17. Composition selon la revendication 15, **caractérisée par le fait que** le polymère cationique est un polymère de polyammonium quaternaire constitué de motifs récurrents répondant à la formule (U) suivante :

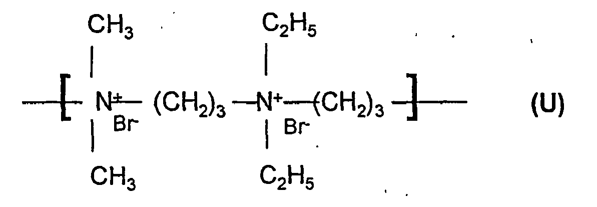

18. Composition selon la revendication 15, **caractérisée par** le fiait que le polymère amphotère est un copolymère comprenant au moins comme monomères de l'acide acrylique et un sel de diméthyldiallylammonium.

19. Composition selon l'une quelconque des revendications 15 à 18, **caractérisée par le fait que** le ou les polymères cationiques ou amphotères représentent de 0,01 % à 10 %, de préférence de 0,05 % à 5 %, et encore plus préférentiellement de 0,1 % à 3 % en poids, du poids total de la composition.

20. Composition selon l'une quelconque des revendications précédentes, **caractérisée par le fait qu'**elle contient au moins un tensioactif choisi parmi les tensioactifs anioniques, cationiques, non ioniques ou amphotères.

21. Composition selon la revendication 20, **caractérisée par le fait que** les tensioactifs représentent 0,01 à 40% et de préférence 0,5 à 30% en poids du poids total de la composition.

22. Composition selon l'une quelconque des revendications précédentes, **caractérisée par le fait qu'**elle contient au moins un agent épaississant additionnel.

23. Composition selon la revendication 22, **caractérisée par le fait que** l'agent épaississant additionnel est un dérivé de cellulose, un dérivé de guar, une gomme d'origine microbienne, un épaississant synthétique.

24. Composition selon la revendication 23, **caractérisée par le fait que** le ou les épaississants additionnels représentent 0,01 à 10% en poids du poids total de la composition prête à l'emploi.

25. Composition selon l'une quelconque des revendications précédentes, **caractérisée par le fait qu'**elle possède un pH allant de 2 à 12.

26. Composition prête à l'emploi selon l'une quelconque des revendications précédentes, **caractérisée par le fait qu'**elle contient en outre un agent oxydant.

27. Composition selon la revendication 26, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

28. Composition selon la revendication 27, **caractérisée par le fait que** l'agent oxydant est le peroxyde d'hydrogène.

29. Composition selon la revendication 28, **caractérisée par le fait qu'**il s'agit d'une solution d'eau oxygénée dont le titre varie de 1 à 40 volumes.

30. Composition selon l'une quelconque des revendications 26 à 29, **caractérisée par le fait qu'**elle possède un pH supérieur à 7 et de préférence supérieur à 8.

31. Procédé de teinture directe des fibres kératiniques et en particulier des fibres kératiniques humaines et plus particulièrement les cheveux, **caractérisé par le fait qu'**il consiste à appliquer sur les fibres une composition de teinture A telle que définie à l'une quelconque des revendications 1 à 25.

32. Procédé de teinture directe éclaircissante des fibres kératiniques et en particulier des fibres kératiniques humaines et plus particulièrement les cheveux, **caractérisé par le fait qu'**il consiste à appliquer sur les fibres une composition résultant du mélange extemporané d'une composition A1 comprenant, dans un milieu approprié pour la teinture, au moins un colorant direct, et d'une composition oxydante B1 comprenant un agent oxydant, la composition A1 et/ou la composition B1 comprenant au moins un polyuréthane associatif cationique tel que défini à l'une quelconque des revendications 1 à 12.

33. Procédé selon les revendications 31 ou 32, **caractérisé par le fait qu'**il consiste à appliquer sur les fibres kératiniques sèches ou humides, la composition prête à l'emploi A ou celle réalisée extemporanément au moment de l'emploi à partir des compositions A1 et B1, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

34. Procédé de teinture directe éclaircissante des fibres kératiniques, en particulier pour fibres kératiniques humaines et plus particulièrement les cheveux, **caractérisé par le fait qu'**il consiste à appliquer sur les fibres kératiniques sèches ou humides, la composition prête à l'emploi réalisée extemporanément au moment de l'emploi à partir d'une composition A' comprenant au moins un colorant direct, mais sans polyuréthane associatif cationique défini aux revendications 1-12, une autre composition A" comprenant au moins un polyuréthane associatif cationique défini selon les revendications 1 à 12, et une composition oxydante B, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

35. Procédé de teinture selon l'une quelconque des revendications 31-34, **caractérisé par le fait que** la composition A ou A1 et/ou la composition B1 comprennent au moins un polymère substantif cationique ou amphotère et au moins un tensioactif.

36. Dispositif à deux compartiments ou " Kit " pour la teinture directe des fibres kératiniques humaines et plus particulièrement les cheveux, **caractérisé par le fait qu'**un compartiment renferme une composition comprenant au moins un colorant direct, et un autre compartiment renferme une composition comprenant au moins un polyuréthane associatif cationique.

37. Dispositifs à deux compartiments ou " Kits " pour la teinture directe éclaircissante des fibres kératiniques humaines et plus particulièrement les cheveux, **caractérisé par le fait qu'**un compartiment renferme une composition A1 comprenant, dans un milieu approprié pour la teinture, au moins un colorant direct, et un autre compartiment renferme une composition B1 comprenant un agent oxydant, au moins un polyuréthane associatif cationique tel que défini à l'une quelconque des revendications 1 à 12 étant présent dans la composition A1 ou dans la composition B1, ou dans chacune des compositions A1 et B1.

## Claims

1. Direct dye composition for keratin fibres, in particular for human keratin fibres and more particularly the hair, comprising, in a medium that is suitable for dyeing, at least one direct dye, said composition being **characterized in that** it also comprises at least one cationic associative polyurethane.

2. Composition according to Claim 1, **characterized in that** the cationic associative polyurethane is of formula (Ia) below:
R-X-(P)ₙ-[L-(Y)ₘ]ᵣ-L'-(P')ₚ-X'-R' (Ia)
in which:
R and R', which may be identical or different, represent a hydrophobic group or a hydrogen atom;
X and X', which may be identical or different, represent a group comprising an amine function optionally bearing a hydrophobic group, or alternatively a group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50 and in particular between 1 and 25,
n, m and p each range, independently of each other, from 0 to 1000;
the molecule containing at least one protonated or quaternized amine function and at least one hydrophobic group.

3. Composition according to Claim 2, **characterized in that** R and R' both independently represent a hydrophobic group, X and X' each represent a group L", n and p are between 1 and 1000, and L, L', L", P, P', Y and m have the meaning given in Claim 2.

4. Composition according to Claim 2, **characterized in that** R and R' both independently represent a hydrophobic group, X and X' both independently represent a group comprising a quaternary amine, n and p are zero, and L, L', Y and m have the meaning given in Claim 2.

5. Composition according to any one of Claims 2 to 4, **characterized in that** R and R' represent a radical or polymer containing a saturated or unsaturated, linear or branched hydrocarbon-based chain, in which one or more of the carbon atoms may be replaced with a hetero atom chosen from S, N, O and P, or a radical or polymer containing a perfluoro or silicone chain.

6. Composition according to any one of Claims 2 to 5, **characterized in that** X and X' represent one of the formulae: 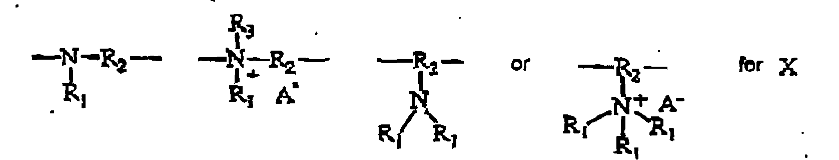 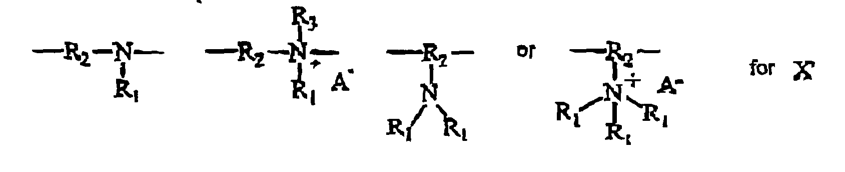 in which:
R₂ represents a linear or branched alkylene radical containing from 1 to 20 carbon atoms, optionally comprising a saturated or unsaturated ring, or an arylene radical, one or more of the carbon atoms possibly being replaced with a hetero atom chosen from N, S, O and P;
R₁ and R₃, which may be identical or different, denote a linear or branched C₁-C₃₀ alkyl or alkenyl radical or an aryl radical, at least one of the carbon atoms possibly being replaced with a hetero atom chosen from N, S, O and P;
A⁻ is a physiologically acceptable counterion.

7. Composition according to any one of Claims 2 to 6, **characterized in that** the groups L, L' and L", which may be identical or different, represent the formula: 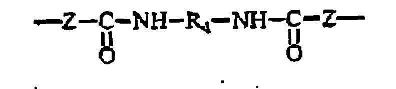 in which:
Z represents -O-, -S- or -NH-; and
R₄ represents a linear or branched alkylene radical containing from 1 to 20 carbon atoms, optionally comprising a saturated or unsaturated ring, or an arylene radical, one or more of the carbon atoms possibly being replaced with a hetero atom chosen from N, S, O and P.

8. Composition according to any one of Claims 2 to 7, **characterized in that** the groups P and P', which may be identical or different, represent at least one of the following formulae: 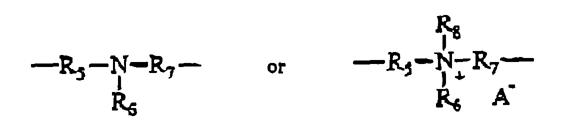 or 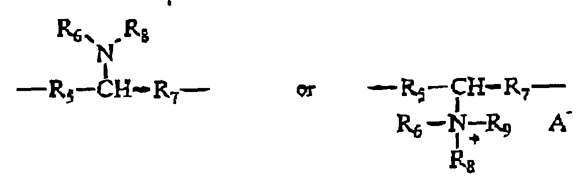 or 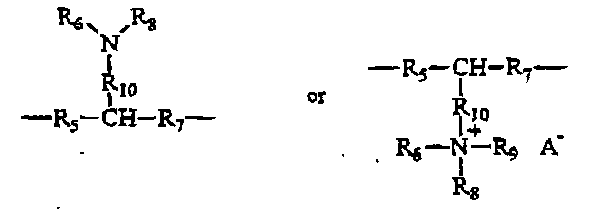 in which:
R₅ and R₇ have the same meanings as R₂;
R₆, R₈ and R₉ have the same meanings as R₁ and R₃;
R₁₀ represents a linear or branched, optionally unsaturated alkylene group possibly containing one or more hetero atoms chosen from N, O, S and P; and
A⁻ is a physiologically acceptable counterion.

9. Composition according to any one of Claims 2 to 8, **characterized in that** Y represents a group derived from ethylene glycol, from diethylene glycol or from propylene glycol, or a group derived from a polymer chosen from polyethers, sulphonated polyesters and sulphonated polyamides.

10. Composition according to any one of the preceding claims, **characterized in that** the cationic associative polyurethanes have a number-average molecular mass of between 400 and 500 000, preferably between 1000 and 400 000 and in particular between 1000 and 300 000.

11. Composition according to any one of the preceding claims, **characterized in that** the cationic associative polyurethanes are used in an amount ranging from 0.01% to 10% by weight relative to the total weight of the composition.

12. Composition according to Claim 11, **characterized in that** the cationic associative polyurethanes are used in an amount ranging from 0.1% to 5% by weight relative to the total weight of the composition.

13. Composition according to any one of the preceding claims, **characterized in that** the direct dye is chosen from neutral, acidic or cationic nitrobenzene direct dyes, neutral, acidic or cationic azo direct dyes, neutral, acidic or cationic quinone, and in particular anthraquinone, direct dyes, azine direct dyes, triarylmethane direct dyes, indoamine direct dyes and natural direct dyes.

14. Composition according to any one of the preceding claims, **characterized in that** the direct dye(s) are present in concentrations ranging from 0.001% to 20% and preferably from 0.005% to 10% by weight relative to the total weight of the composition.

15. Composition according to any one of the preceding claims, **characterized in that** it also contains at least one amphoteric substantive polymer or cationic substantive polymer other than the cationic associative polyurethanes described in any one of Claims 2 to 12.

16. Composition according to Claim 15, **characterized in that** the cationic polymer is a polyquaternary ammonium polymer consisting of repeating units corresponding to formula (W) below:

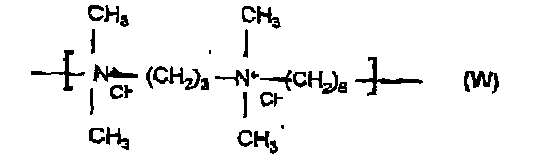

17. Composition according to Claim 15, **characterized in that** the cationic polymer is a polyquaternary ammonium polymer consisting of repeating units corresponding to formula (U) below:

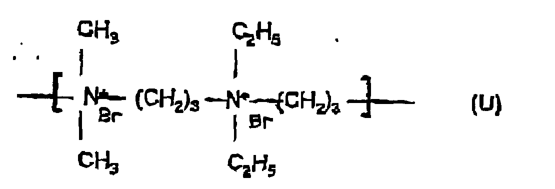

18. Composition according to Claim 15, **characterized in that** the amphoteric polymer is a copolymer comprising at least, as monomers, acrylic acid and a dimethyldiallylammonium salt.

19. Composition according to any one of Claims 15 to 18, **characterized in that** the cationic or amphoteric polymer(s) represent(s) from 0.01% to 10%, preferably from 0.05% to 5% and even more preferably from 0.1% to 3% by weight, relative to the total weight of the composition.

20. Composition according to any one of the preceding claims, **characterized in that** it contains at least one surfactant chosen from anionic, cationic, nonionie and amphoteric surfactants.

21. Composition according to Claim 20, **characterized in that** the surfactants represent from 0.01% to 40% and preferably from 0.5% to 30% by weight, relative to the total weight of the composition.

22. Composition according to any one of the preceding claims, **characterized in that** it contains at least one additional thickener.

23. Composition according to Claim 22, **characterized in that** the additional thickener is a cellulose derivative, a guar derivative, a gum of microbial origin or a synthetic thickener.

24. Composition according to Claim 23, **characterized in that** the additional thickener(s) represent(s) from 0.01% to 10% by weight, relative to the total weight of the ready-to-use composition.

25. Composition according to any one of the preceding claims, **characterized in that** it has a pH ranging from 2 to 12.

26. Ready-to-use composition according to any one of the preceding claims, **characterized in that** it also contains an oxidizing agent.

27. Composition according to Claim 26, **characterized in that** the oxidizing agent is chosen from hydrogen peroxide, urea peroxide, alkali metal bromates or forricyanides, persalts, and redox enzymes, optionally with their respective donor or cofactor.

28. Composition according to Claim 27, **characterized in that** the oxidizing agent is hydrogen peroxide.

29. Composition according to Claim 2B, **characterized in that** it is an aqueous hydrogen peroxide solution whose titre ranges from 1 to 40 volumes.

30. Composition according to any one of Claims 26 to 29, **characterized in that** it has a pH of greater than 7 and preferably greater than 8.

31. Process for the direct dyeing of keratin fibres, in particular human keratin fibres and more particularly the hair, **characterized in that** it consists in applying to the fibres a dye composition A as defined in any one of claims 1 to 25.

32. Process for the lightening direct dyeing of keratin fibres and in particular of human keratin fibres and more particularly the hair, **characterized in that** it consists in applying to the fibres a composition resulting from the extemporaneous mixing of a composition A1 comprising, in a medium that is suitable for dyeing, at least one direct dye, and of an oxidizing composition B1 comprising an oxidizing agent, composition A1 and/or composition B1 comprising at least one cationic associative polyurethane as defined in any one of Claims 1 to 12.

33. Process according to Claim 31 or 32, **characterized in that** it consists in applying the ready-to-use composition A or that prepared extemporaneously at the time of use from compositions A1 and B1 described above, to wet or dry keratin fibres, leaving the composition to act for an action time ranging from 1 to 60 minutes approximately and preferably from 10 to 45 minutes, rinsing the fibres and then optionally washing them with shampoo, followed by rinsing them again and drying them.

34. Process for the lightening direct dyeing of keratin fibres, in particular for human keratin fibres and more particularly the hair, **characterized in that** it consists in applying the ready-to-use composition prepared extemporaneously at the time of use from a composition A' comprising at least one direct dye, but without cationic associative polyurethane defined in Claims 1-12, another composition A" comprising at least one cationic associative polyurethane defined according to Claims 1 to 12, and an oxidizing composition B, to the wet or dry keratin fibres, leaving the composition to act for an action time ranging from 1 to 60 minutes approximately and preferably from 10 to 45 minutes, rinsing the fibres and then optionally washing them with shampoo, followed by rinsing them again and drying them.

35. Dyeing process according to any one of Claims 31-34, **characterized in that** composition A or A1 and/or composition B1 comprise at least one cationic or amphoteric substantive polymer and at least one surfactant.

36. Two-compartment device or kit for the direct dyeing of human keratin fibres, and more particularly the hair, **characterized in that** one compartment contains a composition comprising at least one direct dye, and another compartment contains a composition comprising at least one cationic associative polyurethane as defined in any one of Claims 1 to 12.

37. Two-compartment device or kit for the lightening direct dyeing of human keratin fibres, and more particularly the hair, **characterized in that** one compartment contains a composition A1 comprising, in a medium that is suitable for dyeing, at least one direct dye, and another compartment contains a composition B1 comprising an oxidizing agent, at least one cationic associative polyurethane as defined in any one of Claims 1 to 12 being present in the composition A1 or in the composition B1, or in each of the compositions A1 and B1.

## Patentansprüche

1. Zusammensetzung zum Direktfärben von Keratinfasern, insbesondere menschlichen Keratinfasern und ganz besonders der Haare, die in einem zum Färben geeigneten Medium mindestens einen Direktfarbstoff enthält, **dadurch gekennzeichnet, dass** sie ferner mindestens ein kationisches assoziatives Polyurethan enthält.

2. Zusammensetzung nach Anspruch 1, **dadurch gekennzeichnet, dass** das kationische assoziative Polyurethan die nachstehende Formel (Ia) besitzt
R-X-(P)ₙ-[L-(Y)ₘ]ᵣ-L'-(P')ₚ-X-R' (Ia),
in der bedeuten:
R und R', die gleich oder verschieden sind, eine hydrophobe Gruppe oder ein Wasserstoffatom;
X und X', die gleich oder verschieden sind, eine Gruppe mit einer Aminfunktion, die gegebenenfalls eine hydrophobe Gruppe trägt, oder die Gruppe L";
L, L' und L", die gleich oder verschieden sind, eine von einem Diisocyanat abgeleitete Gruppe;
P und P', die gleich oder verschieden sind, eine Gruppe mit einer Aminfunktion, die gegebenenfalls eine hydrophobe Gruppe trägt; Y eine hydrophile Gruppe;
r eine ganze Zahl von 1 bis 100, bevorzugt von 1 bis 50 und insbesondere von 1 bis 25;
n, m und p jeweils unabhängig voneinander einen Zahlenwert von 0 bis 1 000,
wobei das Molekül mindestens eine protonierte oder quaternisierte Aminfunktion und mindestens eine hydrophobe Gruppe enthält.

3. Zusammensetzung nach Anspruch 2, **dadurch gekennzeichnet, dass** R und R' unabhängig eine hydrophobe Gruppe darstellen, X und X' jeweils eine Gruppe L" bedeuten, n und p einen Zahlenwert von 1 bis 1 000 darstellen und L, L', L", P, P', Y und m die in Anspruch 2 angegebene Bedeutung besitzen.

4. Zusammensetzung nach Anspruch 2, **dadurch gekennzeichnet, dass** R und R' unabhängig eine hydrophobe Gruppe darstellen, X und X' unabhängig eine Gruppe bedeuten, die eine quaternäre Aminfunktion trägt, n und p gleich Null sind und L, L', Y und m die in Anspruch 2 angegebene Bedeutung besitzen.

5. Zusammensetzung nach einem der Ansprüche 2 bis 4, **dadurch gekennzeichnet, dass** R und R' eine Gruppe oder ein Polymer mit einer gesättigten oder ungesättigten, geradkettigen oder verzweigten Kohlenwasserstoffkette, in der eines oder mehrere der Kohlenstoffatome durch ein Heteroatom ersetzt sein können, das unter S, N, O und P ausgewählt ist, oder eine Gruppe oder ein Polymer mit Siliconkette oder einer perfluorierten Kette darstellen.

6. Zusammensetzung nach einem der Ansprüche 2 bis 5, **dadurch gekennzeichnet, dass** X und X' eine der Formeln besitzen: 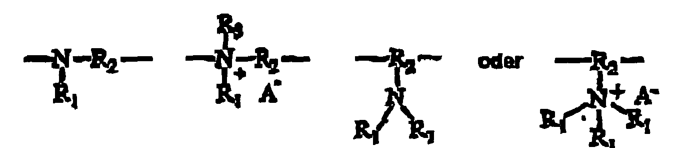 für X 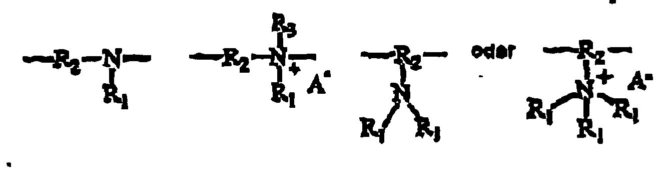 für X'
worin bedeuten:
R₂ eine geradkettige oder verzweigte Alkylengruppe mit 1 bis 20 Kohlenstoffatomen, die gegebenenfalls einen gesättigten oder ungesättigten Ring trägt, oder eine Arylengruppe, wobei eines oder mehrere der Kohlenstoffatome durch ein unter N, S, O und
P ausgewähltes Heteroatom ersetzt sein können;
R₁ und R₃, die gleich oder verschieden sind, eine geradkettige oder verzweigte C₁₋₃₀-Alkylgruppe oder eine geradkettige oder verzweigte C₁₋₃₀-Alkenylgruppe, eine Arylgruppe, wobei mindestens eines der Kohlenstoffatome durch ein unter N, S, O und P ausgewähltes Heteroatom ersetzt sein kann;
A⁻ ein physiologisch akzeptables Gegenion.

7. Zusammensetzung nach einem der Ansprüche 2 bis 6, **dadurch gekennzeichnet, dass** die Gruppen L, L' und L", die gleich oder verschieden sind, die Formel 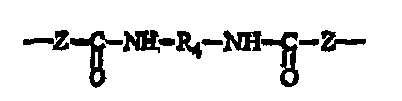 besitzen, in der bedeuten:
Z -O-, -S- oder -NH- und
R₄ eine geradkettige oder verzweigte Alkylengruppe mit 1 bis 20 Kohlenstoffatomen, die gegebenenfalls einen gesättigten oder ungesättigten Ring trägt, eine Arylengruppe, wobei eines oder mehrere der Kohlenstoffatome durch ein unter N, S, O und P ausgewähltes Heteroatom ersetzt sein können.

8. Zusammensetzung nach einem der Ansprüche 2 bis 7, **dadurch gekennzeichnet, dass** die Gruppen P und P', die gleich oder verschieden sind, mindestens eine der nachstehenden Formeln besitzen: 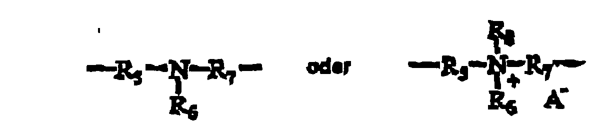 oder 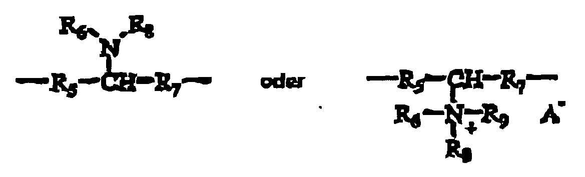 oder 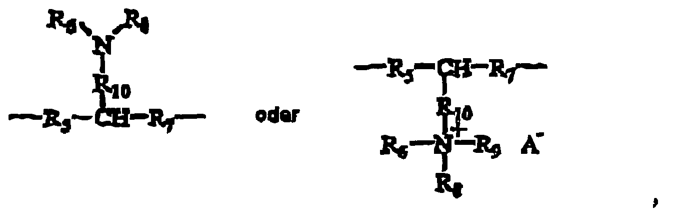 worin bedeuten:
R₅ und R₇ dasselbe wie R₂;
R₆, R₈ und R₉ dasselbe wie R₁ und R₃;
R₁₀ eine geradkettige oder verzweigte, gegebenenfalls ungesättigte Alkylengruppe, die ein oder mehrere Heteroatome enthalten kann, die unter N, O, S und P ausgewählt sind, und
A⁻ ein physiologisch akzeptables Gegenion.

9. Zusammensetzung nach einem der Ansprüche 2 bis 8, **dadurch gekennzeichnet, dass** Y eine von Ethylenglykol, Diethylenglykol oder Propylenglykol abgeleitete Gruppe oder eine Gruppe bedeutet, die von einem Polymer abgeleitet ist, das unter den Polyethern, den sulfonierten Polyeatern und den sulfonierten Polyamiden ausgewählt ist.

10. Zusammensetzung nach einem der vorhergehenden Ansprüche, **dadurch gekennzeichnet, dass** die kationischen assoziativen Polyurethane ein Zahlenmittel der Molekülmasse von 400 bis 500 000, bevorzugt von 1 000 bis 400 000 und insbesondere von 1 000 bis 300 000 besitzen.

11. Zusammensetzung nach einem der vorhergehenden Ansprüche, **dadurch gekennzeichnet, dass** die kationischen assoziativen Polyurethane in einer Menge von 0,01 bis 10 Gew.-% des Gesamtgewichts der Zusammensetzung eingesetzt sind.

12. Zusammensetzung nach Anspruch 11, **dadurch gekennzeichnet, dass** die kationischen assoziativen Polyurethane in einer Menge von 0,1 bis 5 Gew.-% des Gesamtgewichts der Zusammensetzung eingesetzt sind.

13. Zusammensetzung nach einem der vorhergehenden Ansprüche, **dadurch gekennzeichnet, dass** der Direktfarbstoff ausgewählt ist unter den neutralen, sauren oder kationischen direktziehenden Nitrofarbstoffen vom Benzoltyp, neutralen, sauren oder kationischen direktziehenden Azofarbstoffen, den neutralen, sauren oder kationischen Direkdarbstoffen vom Chinontyp und insbesondere den Anthrachinon-Direktfarbstoffen, den direktziehenden Farbstoffen vom Azintyp, den direktziehenden Farbstoffen vom Triarylmethantyp, den direktziehenden Farbstoffen vom Indoamintyp und den natürlichen Direktfarbstoffen.

14. Zusammensetzung nach einem der vorhergehenden Ansprüche, **dadurch gekennzeichnet, dass** der oder die Direktfarbstoffe in Konzentrationen von 0,001 bis 20 Gew.-% und bevorzugt von 0,005 bis 10 Gew.-%, bezogen auf das Gesamtgewicht der Zusammensetzung, vorliegen.

15. Zusammensetzung nach einem der vorhergehenden Ansprüche, **dadurch gekennzeichnet, dass** sie ferner mindestens ein amphoteres oder kationisches substantives Polymer enthält, das von den in den Ansprüchen 2 bis 12 beschriebenen kationischen assoziativen Polyurethanen verschieden ist.

16. Zusammensetzung nach Anspruch 15, **dadurch gekennzeichnet, dass** das kationische Polymer ein quaternäres Polyammonium-Polymer ist, das aus wiederkehrenden Einheiten der nachstehenden Formel (W) besteht:

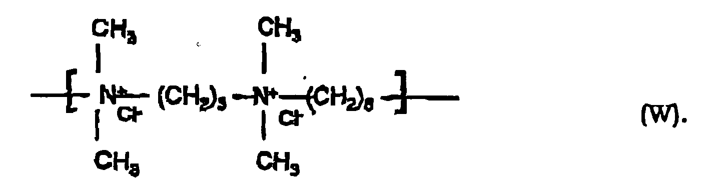

17. Zusammensetzung nach Anspruch 15, **dadurch gekennzeichnet, dass** das kationische Polymer ein quaternäres Polyammonium-Polymer ist, das aus wiederkehrenden Einheiten der nachstehenden Formel (U) besteht:

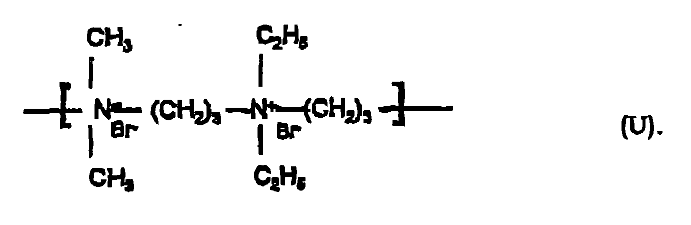

18. Zusammensetzung nach Anspruch 15, **dadurch gekennzeichnet, dass** des amphotere Polymer ein Copolymer ist, das als Monomere mindestens Acrylsäure und ein Dimethyldiallylammoniumsalz enthält.

19. Zusammensetzung nach einem der Ansprüche 15 bis 18, **dadurch gekennzeichnet, dass** das oder die kationischen oder amphoteren Polymeren 0,01 bis 10 Gew.-%, bevorzugt 0,05 bis 5 Gew.-% und noch bevorzugter 0,1 bis 3 Gew.-% des Gesamtgewichts der Zusammensetzung ausmachen.

20. Zusammensetzung nach einem der vorhergehenden Ansprüche, **dadurch gekennzeichnet, dass** sie mindestens ein grenzflächenaktives Mittel enthält, das unter den anionischen, kationischen, nichtionischen oder amphoteren grenzflächenaktiven Mitteln ausgewählt ist.

21. Zusammentsetzung nach Anspruch 20, **dadurch gekennzeichnet, dass** die grenzflächenaktiven Mittel 0,01 bis 40 Gew.-% und bevorzugt 0,5 bis 30 Gew.-% des Gesamtgewichts der Zusammensetzung ausmachen.

22. Zusammensetzung nach einem der vorhergehenden Ansprüche, **dadurch gekennzeichnet, dass** sie zusätzlich mindestens ein Verdickungsmittel enthält.

23. Zusammensetzung nach Anspruch 22, **dadurch gekennzeichnet, dass** das zusätzliche Verdickungsmittel ein Cellulosederivat, ein Derivat von Guargummi, ein Gummi mikrobiellen Ursprungs oder ein synthetisches Verdickungsmittel ist.

24. Zusammensetzung nach Anspruch 23, **dadurch gekennzeichnet, dass** das oder die zusätzlichen Verdickungsmittel 0,01 bis 10 Gew.-% des Gesamtgewichts der anwendungsfertigen Zusammensetzung ausmachen.

25. Zusammensetzung nach einem der vorhergehenden Ansprüche, **dadurch gekennzeichnet, dass** sie einen pH-Wert von 2 bis 12 besitzt.

26. Anwendungsfertige Zusammensetzung nach einem der vorhergehenden Ansprüche, **dadurch gekennzeichnet, dass** sie ferner ein Oxidationsmittel enthält.

27. Zusammensetzung nach Anspruch 26, **dadurch gekennzeichnet, dass** das Oxidationsmittel unter Wasserstoffperoxid, Harnstoffperoxid, Bromaten oder Cyanoferraten(III) von Alkalimetallen, Persalzen und Oxidoreductase-Enzymen, gegebenenfalls mit ihrem entsprechenden Donor oder Cofaktor, ausgewählt ist.

28. Zusammensetzung nach Anspruch 27, **dadurch gekennzeichnet, dass** das Oxidationsmittel Wasserstoffperoxid ist.

29. Zusammensetzung nach Anspruch 28, **dadurch gekennzeichnet, dass** es sich um eine Wasserstoffperoxidlösung handelt, deren Gehalt von 1 bis 40 Volumina variiert.

30. Zusammensetzung nach einem der Ansprüche 26 bis 29, **dadurch gekennzeichnet, dass** sie einen pH-Wert über 7 und bevorzugt einen pH-Wert über 8 besitzt.

31. Verfahren zum Direktfärben von Keratinfasern und insbesondere von menschlichen Keratirdasern und ganz besonders der Haare, **dadurch gekennzeichnet, dass** es darin besteht, auf die Fasern eine Färbezusammensetzung A, wie in einem der Ansprüche 1 bis 25 definiert, aufzubringen.

32. Verfahren zum aufhellenden Direktfärben von Keratinfasern und insbesondere von menschlichen Keratinfasern und ganz besonders der Haare, **dadurch gekennzeichnet, dass** es darin besteht, auf die Fasern eine Zusammensetzung aufzubringen, die durch unmittelbar vor der Anwendung erfolgendes Mischen einer Zusammensetzung A1, die in einem zum Färben geeigneten Medium mindestens einen Direkaarbstoff enthält, und einer oxidierenden Zusammensetzung B1, die ein Oxidationsmittel enthält, resultiert, wobei die Zusammensetzung A1 und/oder die Zusammensetzung B1 mindestens ein kationisches assoziatives Polyurethan, wie in einem der Ansprüche 1 bis 12 definiert, enthält.

33. Verfahren nach Anspruch 31 oder 32, **dadurch gekennzeichnet, dass** es darin besteht, auf die trockenen oder feuchten Keratinfasern die anwenduzigefertige Zusammensetzung A oder die durch unmittelbar vor der Anwendung aus den Zusammensetzungen A1 und B 1 erzeugte Zusammensetzung aufzutragen, sie während einer Verweilzeit von etwa 1 bis 60 Minuten und vorzugsweise 10 bis 45 Minuten einwirken zu lassen, die Fasern zu spülen, sie gegebenenfalls dann mit Shampoo zu waschen und sie anschließend nochmals zu spülen und sie zu trocknen.

34. Verfahren zur aufhellenden Direktfärbung von Keratinfasern, insbesondere von menschlichen Keratinfasern und ganz besonders der Haare, **dadurch gekennzeichnet, dass** es darin besteht, auf die trockenen oder feuchten Keratinfasern die anwendungsfertigte Zusammensetzung aufzubringen, die unmittelbar vor der Anwendung aus einer Zusammensetzung A', die mindestens einen Direktfarbstoff, jedoch kein in den Ansprüchen 1 bis 12 definiertes kationisches assoziatives Polymer enthält, einer weiteren Zusammensetzung A", die mindestens ein in den Ansprüchen 1 bis 12 definiertes kationisches assoziatives Polyurethan enthält, und einer oxidierenden Zusammensetzung B erhalten ist, sie während einer Verweilzeit von etwa 1 bis 60 Minuten und vorzugsweise 10 bis 45 Minuten einwirken zu lassen, die Fasern zu spülen, sie gegebenenfalls dann mit Shampoo zu waschen und sie anschließend nochmals zu spülen und sie zu trocknen.

35. Färbeverfahren nach einem der Ansprüche 31 bis 34, **dadurch gekennzeichnet, dass** die Zusammensetzung A oder A1 und/oder die Zusammensetzung B1 mindestens ein kationisches oder amphoteres aubstantives Polymer und mindestens ein grenzflächenaktives Mittel enthält.

36. Vorrichtung mit zwei Compartments oder "Kit" zum Direktfärben von menschlichen Keratinfasern und insbesondere der Haare, **dadurch gekennzeichnet, dass** ein Compartment eine Zusammensetzung enthält, die mindestens einen Direktfarbstoff enthält, und ein weiteres Compartment eine Zusammensetzung enthält, die mindestens ein kationisches assoziatives Polyurethan, wie in einem der Ansprüche 1 bis 12 definiert, enthält.

37. Vorrichtungen mit zwei Compartments oder "Kit" zur aufhellenden Direktfärbung von menschlichen Keratinfasern und insbesondere der Haare, **dadurch gekennzeichnet, dass** ein Compartment eine Zusammensetzung A1 enthält, die in einem zum Färben geeigneten Medium mindestens einen Direktfarbstoff enthält, und ein weiteres Compartment eine Zusammensetzung B1 enthält, die ein Oxidationsmittel enthält, wobei mindestens ein kationisches assoziatives Polyurethan, wie in einem der Anspruche 1 bis 12 definiert, in der Zusammensetzung A1 oder in der Zusammensetzung B1 oder in beiden Zusammensetzungen A1 und B1 vorliegt.
